# EUROPEAN PATENT APPLICATION

(11) **EP 4 063 344 A1**
(43) Date of publication of application: **28.09.2022**
(21) Application number: 22164476.8
(22) Date of filing: 25.03.2022
(51) Int. Cl.: C07C 51/41, C07C 53/138

(54) **SALT OF 2-ADAMANTYL ACETIC ACID HAVING ANTI-VIRAL ACTIVITY, FORMULATIONS THEREOF AND USES THEREOF**

(30) Priority: 26.03.2021 IT 202100007403
(71) Applicant: Sciamannini, Umbro, 05100 Terni (TR) (IT)
(72) Inventor: Sciamannini, Umbro, 05100 Terni (TR) (IT)
(74) Representative: Metroconsult Srl

(57) **Abstract**

The present invention relates to a compound (a salt) having antiviral activity, which is useful as an active ingredient for use in the sanitizing treatment of ambient air and/or artifacts and/or devices; to a formulation thereof; to a method of producing the same; as well as to a use thereof in a method for sanitizing environments and/or artifacts and/or devices in need of it from the presence of viruses; in particular, the present invention relates to *2-adamantyl cetrimonium acetate* and to the use thereof as an antiviral and/or antifungal and/or antibacterial active ingredient for sanitizing from viruses and/or fungi and/or bacteria environments and/or artifacts and/or devices in need of it.

## Description

### Technical Field of the Invention

The present invention relates to a compound (a salt) having antiviral activity, which is useful as an antiviral active ingredient for use in the disinfection treatment of ambient air and/or environments and/or artifacts and/or devices; to a composition/formulation thereof; to a method of producing the same; as well as to a use thereof in a method for sanitizing air and/or environments and/or artifacts and/or devices in need of it from the presence of viruses and/or fungi and/or bacteria; in particular, the present invention relates to *2-adamantyl cetrimonium acetate* and to the use thereof as an antiviral and/or antifungal and/or antibacterial active ingredient for sanitizing from viruses and/or fungi and/or bacteria air and/or environments and/or artifacts and/or devices in need of it.

### Description and Drawbacks of the Prior Art

A safe-air project within a context of a viral pandemic, e.g., of an unpredictable/unknown type, cannot normally be dealt with, nor conducted and completed, by using substances that are already known in thefield. Indeed, it requires appropriate research that, starting from the study of the molecular behaviour of the virus(es) and/or the variants thereof (e.g., including their identification by sure isolation in a specially equipped laboratory), will lead to creating one or more molecules, and/or compositions thereof, aimed at fighting or reducing/inhibiting the virus(es), and playing their specific role mainly in the air, without however implying any cytotoxicity for the living beings.

The same also applies to the disinfection from viruses of air and/or environments and/or artifacts and/or devices (whether medical or not) in need of it. Merely by way of non-limiting example, some possible mentionable targets are room walls and floors; shops; public places; vehicles; medical devices for individual protection such as gloves, garments, masks; analysis, diagnosis and surgery instruments and equipments; and so forth.

In this respect, the current state of the art is strongly incomplete. In fact, even the WHO found itself without adequate and updated guidelines for sanitizing air and environments as well as for sanitizing artifacts and/or devices as above mentioned. No country on this planet has appropriate guidelines for fighting and limiting recurring pandemics to protect human life. Amplification and transmission of a viral pandemic occurs by people aggregation, frequent travels, high density of human beings in environments with poor air circulation, possible contacts with artifacts and/or devices polluted by the virus. The absence of suitable devices for real-time air control, capable of abating the load of chemical-biological contaminants that can transfer the infection into human cells, and the lack or unclarity of guidelines for ambient air protection, particularly as regards the preventive aspects thereof, i.e., before the air becomes impregnated with polluting agents, significantly increase the risk of pandemic diffusion.

### Technical Problem

The need is therefore still felt in the field for the availability of one or more substances which can provide to a number of suitable formulations an appropriate retroviral activity (i.e., an antiviral activity, as previously described herein), or anyway a *steric energy* as an assembly, in the case of a chemical formulation, of E-torsional, E-pending and E-stretching energy. This sum results in a unique molecular mechanism, because only by providing a preparation with bond orders capable of releasing torsional energy from 80° to 360° in association, i.e., the substance conjugated from two molecules, such preparation will be able to release bond dissociation energy (relative to the heat of formation of the atoms involved); for example, CH bonds have a dissociation energy of about 100 kcal/mol. Such energy sums can be quantistically computed to obtain a dissociation value parameter capable of inhibiting/degrading considerable viral loads in the environment (and/or on artifacts or devices), by identifying an action threshold not below 1.74 - 2.0 logarithms (log). Preferably, reference is made herein to a substance capable of reducing the degree of viral infectivity having an action threshold at least greater than 2 log; more preferably, greater than 2.5 log; even more preferably, greater than 3 log. Though it is important to determine the viruses' degree of infectivity, an indicative logarithmic reduction is determined in suspension assays, since viral persistence is different from bacterial persistence and, de *facto,* never exceeds a presence of virus genome copies of 1.7 log.

It is the object of the present invention to provide an adequate solution to the above-described technical problem.

### Summary of the invention

The present inventor has now surprisingly found that a salt of 2-adamantyl acetic acid with the cetrimonium quaternary ammonium ion (or cetyltrimethylammonium cation) can provide an adequate response to the technical problem highlighted above.

It is, therefore, one object of the present invention the salt 2-adamantyl cetrimonium acetate (or *1-hexadecanaminium, N,N,N,trimethyl-tricyclo[3.3.1.13,7]decane-2-acetate(1:1)),* as disclosed in the appended independent claim.

It is another object of the present invention a composition of the above salt, as disclosed in the appended independent claim.

It is a further object of the present invention a method for preparing the above salt, as disclosed in the appended independent claim.

It is yet another object of the present invention the use of the above salt and/or the composition thereof for disinfecting environments and/or artifacts and/or devices from viruses, as disclosed in the appended claims.

Preferred embodiments of the present invention are disclosed in the appended dependent claims.

### Detailed Description of the Invention

It is therefore the subject of the invention a substance obtained by chemical synthesis which is an easily reactivable pure salt that is capable of providing the desired unique properties.

Said substance of the invention is the salt *2-adamantyl cetrimonium acetate,* or 2-adamantan cetrimonium acetate (or *1-hexadecanaminium, N,N,N,trimethyl-tricyclo[3.3.1.13,7]decane-2-acetate(1:1)*), which has proven to have considerable antiviral activity without being toxic for humans and animals.

Said salt is obtained by reacting *2-adamantyl acetic* (or 2-adamantan acetic) acid compound (C₁₂H₁₈O₂) [VWR: vwr.com, Via San Giusto 85 - 20153 Milan (Italy)] with a quaternary ammonium halide such as, for example, cetrimonium bromide or chloride (cetyltrimethylammonium, or hexadecyltrimethylammonium); preferably, cetrimonium bromide [C₁₉H₄₂BrN].

*2-adamantyl acetic acid* is a known commercially available compound ([VWR: vwr.com, Via San Giusto 85 20153 Milan (Italy)]) that can be generically used in disinfectant compositions.

*Cetrimonium bromide (cetyltrimethylammonium bromide)* is a known commercially available surfactant/detergent quaternary ammonium salt (Lush, Italy) which is commonly used in antiseptic compositions and in skincare and hair-care products.

The novel salt *2-adamantyl cetrimonium acetate* of the present invention is preferably prepared, by way of example, in accordance with one of the following experimental examples.

### Example 1

In a preferred embodiment, 1.2 g of 98% (MW 194.27 g/mol) 2-adamantyl acetic acid are solubilized under agitation in a 50 ml beaker equipped with magnetic stirring at room temperature into a mixture of 10 mL of distilled water and 40 mL of ethyl alcohol for a time of about 10 minutes. In a second beaker, 0.67 g of 97% (MW 364.4 g/mol) cetrimonium bromide are solubilized under magnetic agitation at room temperature into a mixture of 10 mL of distilled water and 40 mL of ethyl alcohol. After solubilization has occurred, the two solutions are mixed together under agitation in one beaker and the desired final salt precipitates. Next, the solvent is slowly evaporated under mild magnetic agitation by mild heating. Alternatively, the solvent can be evaporated by using a rotary evaporator after transferring the final product suspension into a flask. Finally, the solid thus obtained is dried for about two hours in a stove (e.g., a ventilated stove) at about 48 °C.
Final yield: quantitative;
Purity: 98.3-100%.
Molecular formula: C₃₁H₅₉NO₂;
MW: 477.4;
m.p.: 67 °C.

The product obtained was then characterized by Elemental Analysis; ATR Spectroscopy (Dia/ZnSe) [Shimadzu] and turned out to have the expected structure.

### Elemental Analysis (Eager Test Method)

(NHS Analyzer: EA 1500 CHNS-2, Carlo Erba):

| | | | | | | |
|---|---|---|---|---|---|---|
| Calculated (%): | C | 67.50; | H | 10.00; | N | 1.80 |
| Found (%) : | C | 68.51; | H | 9.79; | N | 1.77 |

### Example 2

As an alternative, the novel salt *2-adamantyl cetrimonium acetate* of the present invention is prepared by following the same procedure of Example 1, with the exception that the cetrimonium bromide compound is replaced by a corresponding quantity of cetrimonium chloride compound.

The characterization of the final compound thus obtained is similar to that of the compound obtained in the previous Example 1.

The salt of the invention, as described above, is a solid in powder form at 20°C. Its Density at 20°C is 1000 kg/m³, and its Relative Density is = 1.

As concerns the contained amount of Volatile Organic Compounds (V.O.C.), with reference to Directive 2010/75/UE, this compound showed the following characteristics: V.O.C. content: 0% by weight; V.O.C. density at 20°C: 0 kg/m³ (0 g/L).

Moreover, the salt of the invention, as described above, proved to have no significant toxicity (it could not be classified as harmful in compliance with the CLP Specification of EC Regulation No 1272/2008): for example, its LD50 (oral in mice) turned out to be > 2000 mg/kg.

The salt of the invention, as described above, showed to behave as a steric, torsional and free energy enhancer by extension of the "CH" chain in formulations prepared for the different applications of the corresponding preparations already in use or for new applications, due to the additional property given by the novel salt prepared as an efficacy supplement having a specific retroviral action or as a biological inhibitor of the *Rna-si-ses.*

The method of preparation of the invention made it possible to obtain the elimination of a potentially harmful aspect, i.e., the presence of "Br"/bromide (or "Cl"/chloride); in its turn, the extension of the carbon chain provided kcals useful for attaining a viral and/or fungal and/or bacterial inhibition action greater than 4 log.

The salt is activated by means of a technique of refrigeration at -12 °C after elimination of the hygroscopic portions in a stove followed by a cooling ramp up to a time, experimentally determined in laboratory, of 40 minutes.

The primary synthesis of the salt must be controlled in the elemental analysis as per the expected carbon, hydrogen and nitrogen amounts, with deviations not exceeding 0.2 mg. The salt of the invention has an exclusive use as a basic active agent having an excellent retroviral function, to be used in any kind of chemical formulations approved as medical devices, SMDs (surgical medical devices), sanitizers, as well as therapeutic generic preparations with indirect or direct clinical effects.

In the light of all the above description, a further subject of the present invention consists of a composition comprising, as an antiviral active ingredient, the salt of the invention; said composition being intended for use in the antiviral and/or antifungal and/or antibacterial sanitizing treatment of ambient air and/or environments and/or artifacts and/or devices in need of it. The specified use is preferably realized by preparing a 1N or 2N solution.

In said composition, the salt of the present invention is present in an effective amount of 0.015 g to 100 g relative to a total weight of 100 g of the composition; preferably, from 0.15 g to 100 g; more preferably, from 0.20 g to 100 g; even more preferably, from 0.50 g to 100 g. The maximum value of 100 g of the salt of the present invention obviously refers to the case wherein the composition consists of said salt alone (e.g., packaged as such in bags to be reconstituted in a suitable solvent or carrier (e.g., water or a suitable mixture of water and alcohol)).

The compositions comprising the salt of the present invention as an antiviral active ingredient can be prepared in a manner similar to the traditional disinfecting and/or sterilizing and/or sanitizing pharmaceutical compositions obtained according to the known pharmaceutical preparation techniques. However, the present compositions of the invention may also be formulated, similarly to known compositions for therapeutic use, for use in particular medical applications (by way of non-limiting example, for the treatment of some types of tumors), whether or not together with suitable known substances having pharmacological activity. As a consequence, the said salt of the present invention may, for example, be formulated with effective amounts of co-formulants such as solvents and/or carriers and/or excipients and/or adjuvants and/or preservatives and/or dyes and/or perfumes and/or balsamic compounds and/or biosurfactants and/or degreasers and/or medicinal specialties and/or preparations for topic use, as well as other known formulations for similar or analogous applications in the sanitization field. In addition, as already previously described herein, the salt of the present invention may be provided as such (e.g., as a fine powder or granules) in vacuum-sealed bags, possibly opaque to light, to be reconstituted in a suitable solvent and/or carrier before use. Under particular situations of use (e.g., prolonged use, or use in particular environmental conditions), the salt of the present invention may also be formulated in protected form, e.g., coated with or incorporated into a suitable non-toxic polymer allowing for the controlled (gradual) release thereof over time, thus ensuring a constant concentration of the active principle for all the desired time. Furthermore, the salt of the present invention may also be formulated together with appropriate amounts of other known substances having an antiviral and/or disinfecting and/or antibacterial and/or antifungal activity and so on, so as to attain a broader spectrum of action against the largest possible number of pathogenic and/or potentially pathogenic agents.

The compositions of the salt of the present invention can be formulated and applied in accordance with all the known practices commonly adopted in this technical field. Merely by way of a non-limiting example of the broad range of application of said compositions, they can be formulated as solutions (having variable densities and concentrations as necessary) and applied as coatings (e.g., by spraying or by brush on walls, air-conditioner filters, heating appliances, etc.) and/or in nebulized form (e.g., in/on environments, means of transport, artifacts, garments, protection devices) and/or by dipping instruments and/or devices (e.g., medical devices such as surgical instruments, catheters and various latex devices).

It is, therefore, a further subject of the present invention the use of said salt and/or the compositions thereof for the disinfection treatment from viruses and/or bacteria and/or fungi of ambient air and/or environments and/or artifacts and/or devices (whether medical or not) in need of it.

### Industrial Applicability

The present invention has made it possible to create a novel compound, and compositions thereof, which can be advantageously used for the disinfection treatment from viruses and/or bacteria and/or fungi of ambient air and/or environments and/or artifacts and/or devices (whether medical or not) in need of it.

## Claims

1. The salt compound 2-adamantyl cetrimonium acetate (or *1-hexadecanaminium, N,N,N,trimethyl-tricyclo[3.3.1.13,7]decane-2-acetate(1:1)*)*.*

2. The compound according to claim 1, as an antiviral agent for use in the disinfection treatment from viruses of air and/or environments and/or artifacts and/or devices in need of it.

3. A method for preparing the compound according to claim 1 or 2, comprising at least the following steps:
- reacting the 2-adamantyl acetic acid compound with the cetrimonium bromide or cetrimonium chloride compound;
- evaporating the reaction solvent and then drying the salt precipitate obtained, as described in experimental Examples 1 and 2 of the description.

4. A composition comprising at least an effective amount of 0.015 g to 100 g of the compound according to claim 1 or 2, relative to a total weight of 100 g of the composition.

5. A composition according to claim 4, comprising at least an effective amount of 0.15 g to 100 g of the compound according to claim 1 or 2, relative to a total weight of 100 g of the composition.

6. A composition according to any one of the preceding claims 4 and 5, for use in the disinfection treatment from viruses and/or fungi and/or bacteria of air and/or environments and/or artifacts and/or devices in need of it.

7. A composition according to claim 6, for use in the disinfection treatment from viruses and/or fungi and/or bacteria of air and/or environments and/or artifacts and/or devices in need of it, wherein the inhibition action towards viruses and/or fungi and/or bacteria is greater than 4 log.

8. A composition according to any one of the preceding claims from 4 to 7, further comprising co-formulants, solvents and/or carriers and/or excipients and/or adjuvants and/or preservatives and/or dyes and/or perfumes and/or balsamic compounds and/or biosurfactants and/or degreasers and/or medicinal specialties and/or preparations for topic use.

9. A composition according to any one of the preceding claims from 4 to 8, further comprising other substances that are known to have antiviral and/or disinfectant and/or antibacterial and/or antifungal activity, and to enhance the action against the largest possible number of pathogenic and/or potentially pathogenic agents.
